# EUROPÄISCHE PATENTSCHRIFT

(11) **EP 2 445 472 B1**
(45) Veröffentlichungstag und Bekanntmachung des Hinweises auf die Patenterteilung: **27.12.2017**
(21) Anmeldenummer: 09786446.6
(22) Anmeldetag: 24.06.2009
(51) Int. Cl.: A61K 36/16, A61K 36/185, A61K 36/23, A61K 36/258, A61K 36/31, A61K 36/45, A61K 36/48, A61K 36/63, A61K 36/77, A61K 36/82, A61K 36/886, A61K 8/55, A61K 8/60, A61Q 19/00, A61K 8/97, A61K 9/06, A61K 9/127, A61K 9/19, A61K 31/522, A61K 31/685, A61K 31/716

(54) **ZUSAMMENSETZUNG FÜR KOSMETISCHE, PHARMAZEUTISCHE ODER DIÄTETISCHE ANWENDUNGEN**
COMPOSITIONS FOR COSMETIC, PHARMACEUTIC OR DIETARY APPLICATIONS
COMPOSITIONS POUR DES APPLICATIONS COSMÉTIQUES, PHARMACEUTIQUES OU DIÉTÉTIQUES

(43) Veröffentlichungstag der Anmeldung: 02.05.2012
(73) Patentinhaber: Lipoid GmbH, 67065 Ludwigshafen (DE)
(72) Erfinder: REBMANN, Herbert, 67435 Neustadt (DE)
(74) Vertreter: Reitstötter Kinzebach
(86) Internationale Anmeldenummer: PCT/IB2009/052707
(87) Internationale Veröffentlichungsnummer: WO 2010/150051

(56) Entgegenhaltungen:
- EP-A1- 0 209 037
- EP-A1- 0 556 394
- EP-A1- 0 599 543
- EP-A1- 0 974 332
- WO-A1-00/25606
- WO-A1-86/03938
- WO-A1-96/05808
- WO-A1-97/42936
- WO-A1-2008/078212
- WO-A2-2006/134409
- DE-A1- 1 813 390
- DE-A1- 2 834 308
- DE-A1- 3 239 871
- US-A- 5 783 211
- US-A1- 2006 013 779

## Beschreibung

Die vorliegende Erfindung betrifft eine Zusammensetzung aus pflanzlichem Extrakt und/oder Inhaltsstoffe und/oder Wirkstoffe, Kohlehydrat und Phospholipid, ein Verfahren zur Herstellung einer solchen Zusammensetzung und die pharmazeutische, diätetische oder kosmetische Anwendung einer solchen Zusammensetzung.

Pflanzliche Rohstoffe spielen bei der Zubereitung von pharmazeutischen, diätetischen und kosmetischen Anwendung eine immer bedeutendere Rolle. Die Nachfrage nach rein natürlichen Formulierungen ist von steigender Bedeutung. Pflanzliche Rohstoffe, insbesondere pflanzliche Extrakte mit wirksamen Bestandteilen sind nicht immer stabil und in der Regel nicht leicht in eine Formulierung einzuarbeiten.

In der US 5,387,415 werden Aloe vera juice pellets hergestellt, indem der Saft mit Collagenen in flüssigen Stickstoff bei -196°C getropft wird. Das Verfahren ist sehr arbeitsintensiv und führt zu hohem Energieverbrauch. Ein ähnliches Verfahren ist in US 5,401,502 beschrieben.

US 5,783,211 beschreibt Bisabolol Nanopartikel, Preemulsion mit Lecithin im Hochdruckhomogenisator, anschließend mit Stärke und Maltodextrin sprüh-getrocknet. Es muss immer mit einer Stärke Matrix gearbeitet werden. Die Produkte werden sprühgetrocknet. Viele pflanzliche Extrakte und deren Inhaltsstoffe sind sehr empfindlich und können bei der Sprühtrocknung zersetzt bzw. abgebaut werden, was vermieden werden sollte.

US 5,180,713 beschreibt die Herstellung von pharmazeutischen Liposomen in organischen Lösungsmitteln in Gegenwart von Kryoprotektoren, wie z.B. Zuckern. Es muss in organischen Lösungsmitteln gearbeitet werden, die sich schwer aus dem Endprodukt entfernen lassen und in natürlichen Produkten nicht erwünscht sind.

US 6,534,087 beschreibt aufgeschäumte Tabletten aus Wirkstoff; Lecithin und Maltodextrin. Hieraus lassen sich keine dosierbaren Pulver oder Granulate mit stabilen Eigenschaften herstellen.

US 20040234673 offenbart eine Zusammensetzung mit einer amorphen Kohlehydratphase, einer kristallinen Phase und einer dritten Phase, die aus einem oder mehreren Stoffen ausgewählt ist, die aus Aromen, flüchtigen Stoffen und Stoffen, die empfindlich sind gegen äußere Einflüsse, gebildet sind, wobei die 3. Phase in den andern beiden Phasen mittels Emulgator dispergiert ist. Die Extrusion dieser Zusammensetzung erfolgt bei hohen Temperaturen. Durch das Arbeiten bei höheren Temperaturen werden die Inhaltsstoffe von Pflanzenextrakten zersetzt und können ihre gewünschte Aktivität nicht entfalten.

In den Patentanmeldungen EP 209037, EP 209038, EP 275005, EP 275224,
EP 283713, EP 300282, EP 304603, EP 441279, EP 464297, EP 1390008,
EP 1837030, EP 1844785 werden Pflanzenextrakte stabilisiert durch Komplexbildung der Wirkstoffe mit Phospholipiden; hierzu muss in Lösungsmitteln, wie z.B. Methylenchlorid oder Methanol, gearbeitet werden. Die Arbeitsweise ist sehr aufwendig und bedingt die Verwendung von bedenklichen Lösungsmitteln.

Die Probleme bei der Formulierung und die begrenzte Stabilität bei Transport und Lagerung der Zusammensetzungen des genannten Stands der Technik sind auf die Auswahl von deren Komponenten zurückzuführen, insbesondere z. B. der Qualität der Lecithine mit Phosphatidylcholingehalten von weniger als 15 %.

Der vorliegenden Erfindung liegt deshalb die Aufgabe zugrunde, eine Zusammensetzung bereitzustellen, die einen hohen Anteil an pflanzlichem Extrakt und/oder Inhaltsstoffen und/oder Wirkstoffen hat, hohe Stabilität bei Transport und Lagerung aufweist und sich leicht und schonend in Formulierungen verarbeiten lässt, ein Verfahren zur Herstellung einer solchen Zusammensetzung zu schaffen und geeignete Anwendungen einer solchen Zusammensetzung.

Die Lösung erfolgt mit Zusammensetzungen mit den Merkmalen des Anspruchs 1, einem Verfahren zur Herstellung einer solchen Zusammensetzung mit den Merkmalen des Anspruchs 6 und mit Anwendungen mit den Merkmalen des Anspruchs 7.

Die erfindungsgemäßen Zusammensetzungen enthalten 28-32 % pflanzlichen Extrakt und/oder pflanzlichen Wirkstoff, 60-70 % Maltodextrin und 2-8 % Phospholipid aus Phospholipid-Fraktionen mit einem Phosphatidylcholine-Gehalt von 40-80 %. Bevorzugt sind Zusammensetzungen mit 30 % pflanzlichem Extrakt und/oder pflanzlichem Wirkstoff, 67 % Maltodextrin und 3 % Phospholipid aus Phospholipid-Fraktionen mit einem Phosphatidylcholine-Gehalt von 40-80 %. Die Zusammensetzungen erlauben es, hohe Wirkstoffgehalte in den Formulierungen zu erzielen. Die Zusammensetzungen sind konservierungs- und lösungsmittelfrei und bestehen weitestgehend nur aus natürlichen pflanzlichen Rohstoffen.

Als pflanzlicher Rohstoff kommen Extrakt aus Pflanzen oder Pflanzenteilen, Fruchtkonzentraten, Kräuterextrakten, pflanzliche Öle und dergleichen in Frage wie z.B. Acai-Extrakt (Euterpe oleracea), Acerolaextrakt (Malpighia glabra), Acker-Schachtelhalm (Equisetum arvense, Zinnkraut), Agariusextrakt (Agarius blazei murill), Aloe (Aloe vera, Aloe Barbadensis), Apfelextrakt (Malus), Artischokenblätterextrakt (Cynara scolymus), Artischockenblütenextrakt (Cynara edulis), Arnika (Arnica Montana), Austernextrakt, Ostrea edulis), Baldrianwurzelextrakt (Valeriana officinalis), Bärentraubenblätterextrakt (Artostaphylos uva-ursi), Bambusextrakt (Bambus vulgaris, Bamboo), Bittermeloneextrakt(Momordica charantia), Bitterorangeextrakt (Citrus aurantium), Brennesselblätterextrakt (Urtica dioica), Brennnesselwurzel-Extrakt (Urtica dioica), Brokkoliextrakt (Brassica oleracea), Brunnenkresse (Rorippa nasturtium), Buntnesselextrakt (Coleus forskohlii), Capsicumextrakt (Capsicum frutescens), Centella asiatica (Gotu Kola), Chinarindenextrakt (Cinchona), Cranberryextrakt (Vaccinium vitis-daea), Curcumaextrakt (Curcuma longa), Damianaextrakt (Tunera diffusa), Drachenfruchtextrakt (Pitahaya), Echinacea Purpurea, Weizen Plazenta Extrakt, Edelweissextrakt (Leotopodium alpinum), Efeuextrakt (Hedera helix), Erd-Wurzeldornextrakt (Tribulus terrestris), Garcinia Cambogiaextrakt (Garcinia Cambogia), Ginkgoextrakt (Ginkgo biloba), Ginsengextrakt (Panax ginseng), Granatapfelextrakt (Punica granatum), Grapefruitextrakt (Citrus paradisi), Griffoniaextrakt (Griffonia simplicifolia), Grüner Tee Extrakt (Camellia sinensis), Guaranaextrakt (Paullinia cupana), Gurkenextrakt (Cucumis sativus), Hagebuttenextrakt (Rosa canina), Heidelbeerextrakt (Vaccinium myrtillus), Hibiskusextrakt (Malvacea), Malvenextrakt, Honigextrakte, Hopfenextrakt (Humulus), Ingwerextrakt (Zingiber officinale), Isländische Moose (Ceteraria islandica), Jojobaextrakt (Simmondsia chinensis), Johanniskraut (St. Johns Wort, Hypericum Perforatum), Kaffee Konzentrat, Kakaobohnenextrakt (Theobroma cacao), Kaktusblütenextrakt, Kamillenblütenextrakt (Matricaria recutita, Chamomile, Matricaria Chamomila), Karottenextrakt (Daucus carota), Kiwiextrakt (Aperygidae), Kudzuextrakt (Pueraria lobata), Kokosnussmilchextrakt, Kürbiskernextrakt (Curcurbita pepo), Kornblumenextrakt (Centaurea cyanus), Lotusblumenextrakt, Löwenzahnwurzelextrakt (Taraxacum officinale), Macaextrakt (Lepidium peruvianum), Magnolienblütenextrakt, Mangoextrakte, Mariendistelextrakt (Silybum marianum), Mariengold (Calendula Officiennalis), Mateextrakt (Hex paraguariensis), Mäusedornextrakt (Rugcus aculeatus), Meeresalgenextrakte, Moosbeere (Cranberry, Kraanbeere, Vaccinium macrocarpon), Moringa Oleiferaextrakt, Moschus Malve (Malva moschata), Nachtkerzenölextrakt (Azadirachta indica), Nesselextrakt (Urticaceae), Olivenblätterextrakt (Olea europea), Orangenextrakt (Hespridin), Orchideenextrakt, Papayaextrakt (Carica papaya), Pfefferminzextrakte, Carica papaya (Geissospermum), Pomeranzextrakt (Citrus aurantioum), Preiselbeerenextrakt (Vaccinium vitas-ideea), Pygeum Afrikanum Extrakt (Prunus africana), Rebenkrautextrakte, Resveratrolextrakt (Polygonum cuspidatum), Rooibos (Aspalasthus Linnearis), Rosenblütenextrakt, Rosskastanienextrakt (Aesculus hippocastanum, Horse chestnut), Rosmarin (Rosmary, Rosemarinus Officinalis), Rotklee Extrakt (Trifolium platense), Rotweinextrakt (Vitis vinifera), Sägepalmenextrakt (Serenoa repens), Salatextrakt (Lactuca sativa), Sandelholzextrakt (Santalum rubrum), Salbei (Sage, Salvia Officinalis), Schachtelhalmextrakt (Equisetum), Schafgarbenextrakt (Achillea millefolium), Schwarzer Pfeffer Extrakt (Piper nigrum), Schwarzteeextrakt, Seerosenextrakt (Nymphaea), Silberweide, (Willow Bark, Salix Alba), Süssholz (Glycyrrhiza), Teufelskrallenwurzelextrakt (Harpagophytum procumbens), Thymianextrakt (Thymus vulgaris), Tomatenextrakt (Lycopersicum esculentum), Traubenkernextrakt (Vitis vinifera), Traubenschalenextrakt (Vitis vinifera), Wasserkresse (Rorippa amphibia), Weidenrinden-Extrakt (Salix alba), Weihrauchextrakt (Artemisia absinthium), Weissteeextrakt, Yamswurzelextrakt (Dioscorea opposita), Yohimbinextrakt (Pausinystalia yohimbe), Zaubernuss (Hamamelis), Zimtrindenextrakt (Cinnamomum cassia Presl), Zitronenextrakt (Citrus), Zwiebelextrakt (Allium cepa).

Neben den Extrakten können auch pflanzliche Wirk- oder Inhaltsstoffe allein oder in Kombination mit den Extrakten verwendet werden. Als pflanzliche Wirkstoffe kommen z.B. in Frage: Glycyrrhizin , Koffein, Proanthocianidin, Hesperitin, Rutin, Luteolin, Polyphenole, Aspalatin, Oleuropin, Theobromin, Bioflavanoide oder Kombinationen aus Glycyrrhizin und Glycerica glabra Extrakten, Koffein und Camellia sinesis oder Camellia alba oder Guarana (Paulinia cubana) oder Coffes arabica, Proanthocianidin und Vitis vinifera (Weinrebe), Hespiridin und Rutin und Citrus aurantii amara Schalenextrakt, Luteolin und Chamomilla reticulataq, Polyphenole und Camellia sinensis oder Vaccinium macrocarpon (Cranberry), Koffein/Theobromin und Hex paraguaiensis (Mate), Biovlavanoide und Grünteeextrakt (Camellia sinensis), Curcumin und Curcuma longa, Epigallocatechingallate, Catechin, Epicatechine, Narginin, Hesperidin, Neohesperidin, Asiaticiside, Ellagitannin, Chlorogensäure, Oleuropei, Arbutin, Betuli, Esculin, Sylimarin, etc., insbesondere Isoflavone Puerin (aus oder mit Pueraria lobata Extrakt), Oleuropein (aus oder mit Olea europea Leaf extrakt), Luteolin (aus oder mit Chamilla reticulate), Baicalin (aus oder mit Scutellaria balcalinensis Rott extrakt, Chorogenic acid (aus oder mit Eucommia ulmoides/Guttaperchabaum Extrakt), Apigenin (aus oder mit Citrus grandis peel extract), Hesperidin (aus oder mit Citrus aurantium amara peel extract), Coffein (aus Paulinia cubana/Guarana Extrakt), Aspalatin (aus oder mit Aspalatus linnearis/Rooibos Extrakt), Rutin (aus oder mit Sophora japonica Extrakt), Polyphenol, Quinic acid (aus oder mit Vaccinium marcrocarpon/Cranberry Extrakt), Allagitannins (aus oder mit Punica granatum/Pomegranate Extrakt), Asiaticoside (aus oder mit Centella asiatica Extrakt), Proanthocianidine (aus oder mit Vitis vinifera Extrakt (Proanthocianidine)), Rutin (aus oder mit Viola odorata Extrakt), 18 beta Glycyrrhetic acid (aus oder mit Liquiritia Extrakt), Glycyrrhizin (aus oder mit Liquiritia Extrakt), Theanin, Polaphenols (aus oder mit Camellia/Thea sinsnesis Extrakt), Coffeolychinic acid, Caffeine, Theobromone (aus oder mit Hex paraguaiensis/Mate Extrakt).

Als Phospholipide kommen Phospholipide und Phospholipid-Fraktionen aus pflanzlichen Rohstoffen wie z.B. aus Sojabohnen, Sonnenblumen, Raps, Erdnuss, Mais, Lupinen oder Baumwollsaat oder Eigelbphospholipide mit einem Phosphatidylcholine-Gehalt von 40-80 % oder definierte Phospholipide wie Di-acylphosphatidylcholine (wie z. B. Dimyristoyl-, Dipalmitoyl-, Disteraoylphosphatidylcholin), hydrierte Phospholipide, hydrolisierte oder modifizierte Phospholipide mit einem Phosphatidylcholine-Gehalt von 40-80 % in Frage.

Lecithin setzt sich aus Neutralipiden, Glycolipiden und Phospholipiden zusammen. Die Phospholipide setzen sich zusammen aus z.B. Phosphatidylcholin (PC), Phosphatidylethanolamin (PE), Phosphatidylinositol (PI), Phosphatidylserin (PS), Lysophosphatidylcholine (LPC), Lysophosphatidylethanolamin (LPE), Lysophosophatidylinositol (LPI) usw. (A. Wendel Lecithin, Kirk-Othmer Encyclopedia of Chemical Technology, Fourth Edition, Volume 15; Willem van Nieuwenhuyzen, Fett/Lipid 99 (1997), Nr. 1, S10-14, Willem van Nieuwenhuyzen, Eur. J. Lipid Sei. Technol. (2008), 472-486. Handelsübliches Lecithin enthält ca. 12-15 % Phosphatidylcholin. Entöltes Lecithin, auch Reinlecithin genannt, enthält 20-25 % Phosphatidylcholin. Durch Fraktionierung mit Ethanol lassen sich Phospholipid-Fraktionen mit 30 bis 100 % Phosphatidylcholin Gehalt herstellen. Phosphatidylcholin angereicherte handelsübliche Produkte sind z.B. Phospholipide aus Soja wie z.B. Lipoid S 20 (ca. 20-24 %PC, 16-22 %PE), Lipoid S45 (45 %PC, 10-18 %PE), Lipoid S75 (70 %PC, 7-10 %PE), Lipoid S80 (75 %PC, 7 %PE), Lipoid SIOO (> 94 %PC), Phospholipon 20 (ca. 20-24 %PC, 16-22 %PE), Phospholipon 50 (ca. 45-50 %PC), Phospholipon 85G (> 80 % PC), Phospholipon 9OG (> 95 %PC) oder Phospholipide aus Raps, wie z.B. Lipoid R20 (ca. 20-24 %PC, 16-22 %PE), Lipoid R75 (70 %PC, 7-10 %PE), Lipoid R45 (45 %PC, 10-18 %PE), Lipoid R75 (75 %PC, 7%PE), Lipoid R80 (75 %PC, 7 %PE), Lipoid R100 (> 94 %PC) oder Soja Phospholipide aus nicht genetisch modifizierten (non GMO) Sojabohnen wie z.B. Lipoid P45 (45 %PC, 10-18 %PE), Lipoid P75 (75 %PC, 7%PE), Lipoid P100 (> 94 %PC).

Als Kohlehydrat wird Maltodextrin eingesetzt.

Die Herstellung der erfindungsgemäßen Zusammensetzungen erfolgt indem die Komponenten gleichzeitig oder nacheinander bei Raumtemperatur in Wasser gelöst bzw. dispergiert werden und in zur Homogenisierung üblichen Geräten, wie z.B. mittels Microfluidizer, 1 bis 3 Durchläufe, homogenisiert, danach filtriert oder steril filtriert, z. B. mittels 2-8 µm Filterkerzen, und anschließend schonend bei -30 °C bis +45 °C gefrier- oder Vakuum getrocknet werden. Das getrocknete Produkt kann mit entsprechenden Geräten bei einem Lochdurchmesser von 0,5-1,5 mm auf die gewünschte Korngröße gemahlen werden.

Beim Redispergieren lassen sich stabile Liposomal Präparate herstellen.

Die getrocknete Zusammensetzung ist Transport- und Lagerstabil und lässt sich leicht in kosmetische, diätetische und pharmazeutische Formulierungen einarbeiten. Vorteile sind die gute Löslichkeit, die Benetzbarkeit des Wirkstoffes wird verbessert, das Dispergierverhalten des Wirkstoffes wird optimiert, die Stabilität des Wirkstoffes gegen Umwelteinflüsse wird verbessert, der Geruch und Geschmack des Wirkstoffes wird optimiert. Bei der Verarbeitung der neuen Zusammensetzungen in kosmetischen, diätetischen oder pharmazeutischen Formulierungen wird die optimale Bioverfügbarkeit erreicht. Formulierungen sind z.B. oral, wie Pulver Granulate, Tabletten, Kapseln, und topische Präparate wie Cremes, Lotion etc., unter Zusatz der üblichen Hilfsstoffe.

### Beispiele:

### Beispiel 1 (nicht erfindungsgemäß)

Aloe Vera 10 kg (10 %) werden mit 87 kg (87 %) Maltodextrin und 3 kg (3 %) Lipoid P45 gemischt und in 100 kg Wasser dispergiert und anschließend mittels eines Microfluidizer 2x homogenisiert. Die Dispersion wird dann über eine 0,2 µm Filterkerze unter Laminarflow sterilfiltriert, bei -30°C eingefroren und im Vakuum bei -35 °C bis maximal +45 °C getrocknet.

Nach der Trocknung werden die Agglomerate gemahlen.

### Beispiel 2

Weißteeextrakt: 30 %
Maltodextrin: 67 %
Lipoid P 45: 3 %

Die Herstellung erfolgt analog Beispiel 1.

### Beispiel 3

Grünteeextrakt: 30 %
Maltodextrin: 67 %
Lipoid P 45: 3 %

Die Herstellung erfolgt analog Beispiel 1.

### Beispiel 4

Hibiscusextrakt: 30 %
Maltodextrin: 67 %
Lipoid P 45: 3 %

Die Herstellung erfolgt analog Beispiel 1.

### Beispiel 5

Guaranaextrakt: 30 %
Maltodextrin: 67 %
Lipoid P 45: 3 %

Die Herstellung erfolgt analog Beispiel 1.

### Beispiel 6

Zitronensäure: 15 %
Weinsäure: 10 %
Milchsäure: 5 %
Aerosil: 5 %
Maltodextrin: 62 %
Lipoid P45: 3 %

Die Herstellung erfolgt analog Beispiel 1.

### Beispiel 7 (nicht erfindungsgemäß)

Weißteeextrakt: 20 %
Maltodextrin: 70 %
Lipoid S20: 10 %

Die Herstellung erfolgt analog Beispiel 1.

### Beispiel 8 (nicht erfindungsgemäß)

Grünteeextrakt (mit 1% Koffein und 5% Polyphenolen): 40 %
Threhalose: 59 %
Lipoid S75: 1 %

Die Herstellung erfolgt analog Beispiel 1.

### Beispiel 9 (nicht erfindungsgemäß)

Guaranaextrakt: 20 %
Glucose: 78 %
Phospholipon 80: 2 %

Die Herstellung erfolgt analog Beispiel 1.

### Beispiel 10 (nicht erfindungsgemäß)

Weißteeextrakt: 28 %
Mannit: 70 %
Lipoid S80: 2 %

Die Herstellung erfolgt analog Beispiel 1.

Bevorzugt sind kosmetische Zubereitungen.

### Beispiel 11

### Herstellung einer Soft Creme

Phase A: 40,0 g SLM 2005 (SLM 2005 = Lipoid SLM 20 Basisformulierung bestehend aus hydriertem Sojalecithin, Ethanol, Glycerin und mittelkettigen Triglyceriden).
Phase B: 82,8 g destilliertes Wasser, 0,2 g Keltrol CGG-SFT (Xanthangummi Produkt mit laminarer-fließ-Rheologie für transparente Lösungen, die insbesondere ausgelegt sind zur Verwendung bei kosmetischen und weiteren Körperpflegeprodukten. Staubfreies, dünnflüssiges Puder, das stabil ist über einen großen pH Bereich), 1,0 g Phospholipon 80H, 1,0 g Zusammensetzung gemäß Beispiel 1-5
Phase C: 36,0 g Miglyol, 12,0 g Jojobaöl, 2,0 g Vitamin-E-acetat
Phase D: 12,0 g Ethanol, 6,0 g Glycerin, 6,0 g Hydrolite, 1,0 g Panthenol

Die Phase B wird auf 60-65 °C erwärmt bis alle Bestandteile gelöst sind und anschließend unter Rühren bei 40 °C zu Phase A eingearbeitet, danach Phase C eingerührt und anschließend danach wird Phase D zugegeben unter Rühren ca. 1 Minute am Ultra-Turax bei 40 °C homogenisiert.

### Beispiel 12

### Herstellung einer Soft Creme

Phase A: 40,0 g SLM 2005
Phase B: 77,8 g destilliertes Wasser, 0,2 g Keltrol CG-SFT, Phospholipon 80H
Phase C: 36,0 g Miglyol, 12,0 g Jojobaöl, 2,0 g Tocopherolacetat
Phase D: 12,0 g Ethanol, 6,0 g Glycerin, 6,0 g Hydrolite, 1 g Panthenol
Phase E: 5,0 g destilliertes Wasser, 1,0 g der Zusammensetzung gemäß Beispiel 1-5.

Die Phase B wird auf 60-65 °C erwärmt bis alle Bestanteile gelöst sind und anschließend unter Rühren bei 40 °C zu Phase A eingearbeitet; danach wird Phase C unter Rühren zugegeben und homogenisiert; danach wird die bei 400 °C homogenisierte, Phase D zugegeben und unter Rühren ca. 1 Minute am Turax bei 40 °C homogenisiert.

Danach wird unter Kaltrühren die Phase E zugegeben.

### Beispiel 13

### Herstellung einer Lotion

Phase A: 20,0 g SLM 2005
Phase B: 107,6 g destilliertes Wasser, 0,4 g Keltrol CGG-SFT = Xanthan Gum (s. o.), 3,0 g Phospholipon 80H, 1,0 g Zusammensetzung gemäß Beispiel 1-5, 1,0 g Panthenol
Phase C: 30,0 g Miglyol, 6,0 g Jojobaöl, 2,0 g Tocopherolacetat
Phase D: 14,0 g Ethanol, 106,0 g Glycerin, 6,0 g Hydrolite

Die Phase B wird auf 60-65°C erwärmt bis alle Bestanteile gelöst sind und anschließend unter Rühren bei 40 °C zu Phase A eingearbeitet; danach wird Phase C zugegeben und homogen gerührt und anschließend die Phase D zugegeben und unter Rühren ca. 1 Minute am Ultra-Turax bei 40 °C homogenisiert.

### Beispiel 14

### Herstellung einer Lotion

Phase A: 20,0 g SLM 2005
Phase B: 97,6 g destilliertes Wasser, 0,4 g Keltrol CGG-SFT,
   3,0 g Phospholipon 80H = hydriertes Phospholipid mit ca. 80 % Phosphatidylcholine, 1,0 g Zusammensetzung gemäß Beispiel 1-5, 1,0 g Panthenol
Phase C: 30,0 g Miglyol = Fettsäureester, 6,0 g Jojobaöl, 1,0 g Tocopherolacetat, 14,0 g Ethanol, 10,0 g Glycerin, 6,0 g Hydrolite,
Phase D: 10,0 g destilliertes Wasser, 1,0 g der Zusammensetzung gemäß Beispiel 1-5.

Die Phase B wird auf 60-65 °C erwärmt bis alle Bestanteile gelöst sind und anschließend unter Rühren bei 40 °C zu Phase A eingearbeitet, danach wird Phase C zugegeben und homogen gerührt und anschließend die Phase D zugegeben und unter Rühren ca. 1 Minute am Ultra-Turax bei 40 °C homogenisiert.

### Beispiel 15

### Creme

Phase A: 18,0 g Miglyol 812, 9,0 g Tegosoft DC, 10,0 g Avocadoöl, 6,0 g Lanette 18, 2,0 g Tegin M, 0,2 g Stearinsäure, 1,0 g Tocopherolacetat
Phase B: 2,0 g TegoCare Cg 90; 6,0 g Glycerin, 1,0 g Panthenol, 0,4 g Allatoin, 132,2 g Wasser
Phase C: 1,0 g der Zusammensetzung gemäß Beispiel 1-5, 9,0 g Wasser
Phase D: 0,4 g Konservierungsmittel.

Phase A und B werden jeweils auf 80 °C erwärmt. Phase B wird in Phase A unter Rühren eingearbeitet und anschließend 1 Minute mit einem Ultra-Turex nachhomogenisiert. Beim anschließenden Kaltrühren wird Phase C und D zugerührt.

## Patentansprüche

1. Zusammensetzung aus pflanzlichem Wirkstoff oder pflanzlichem Extrakt, Maltodextrin und Phospholipid, **dadurch gekennzeichnet, dass** die Zusammensetzung in trockener Form als Pulver oder Granulat vorliegt und dass
a. 28 - 32% pflanzlicher Wirkstoff oder pflanzlicher Extrakt,
b. 60 - 70% Maltodextrin und
c. 2 - 8% Phospholipid aus Phospholipid-Fraktionen mit einem Phosphatidylcholin-Gehalt von 40-80% enthalten sind.

2. Zusammensetzung gemäß Anspruch 1, **dadurch gekennzeichnet, dass**
a. 30% pflanzlicher Wirkstoff oder pflanzlicher Extrakt,
b. 67% Maltodextrin und
c. 3% Phospholipid enthalten sind.

3. Zusammensetzung gemäß Anspruch 1 oder 2, **dadurch gekennzeichnet, dass** die trockne Form ein Lyophilisat ist.

4. Zusammensetzung gemäß einem der Ansprüche 1-3, **dadurch gekennzeichnet, dass** als pflanzlicher Extrakt ein Extrakt aus Fruchtkonzentraten, Kräuterextrakte oder pflanzliche Öle vorgesehen sind.

5. Zusammensetzung gemäß einem der Ansprüche 1-4, **dadurch gekennzeichnet, dass** als pflanzliche Wirkstoffe oder pflanzliche Extrakte Aloe (Aloe vera), Rooibos (Aspalathus Linnearis), Guarana (Paullinia cupana), weißer Tee, grüner Tee, schwarzer Tee, Hibiskus, Nessel (Urticaceae), Wasserkresse, Brunnenkresse, Schachtelhalm, Zinnkraut, Kamille, Centella asiatica (Gotu Kola), Ginggo, Ginseng, Moosbeere (Cranberry, Kraanbeere, Vaccinium macrocarpon) Olive, Granatapfel oder Mischungen und/oder Extrakte derselben vorgesehen sind.

6. Verfahren zur Herstellung von Zusammensetzungen gemäß einem der Ansprüche 1-5, **dadurch gekennzeichnet, dass** die Komponenten in Wasser gelöst bzw. dispergiert und homogenisiert werden, danach vorzugsweise steril filtriert und anschließend bei - 30°C bis + 45°C gefrier- oder vakuumgetrocknet und das getrocknete Produkt auf eine Korngröße mit einem Lochdurchmesser von 0,5 - 1,5mm gemahlen wird.

7. Verwendung von Zusammensetzungen gemäß einem der Ansprüche 1-5 zur Herstellung von kosmetischen, diätetischen oder pharmazeutischen Formulierungen.

## Claims

1. A composition of vegetable active substance or vegetable extract, maltodextrin and phospholipid, **characterized in that** the composition is provided in a dry form as a powder or as granules and that it contains:
a. 28 - 32 weight % of vegetable active substance or vegetable extract;
b. 60 - 70 weight % of maltodextrin; and
c. 2 - 8 weight % of phospholipid from phospholipid fractions having a phosphatidyl choline content of 40 - 80 %.

2. The composition according to claim 1, **characterized in that** it contains:
a. 30 weight % of vegetable active substance or vegetable extract;
b. 67 weight % of carbohydrate; and
c. 3 weight % of phospholipid.

3. The composition according to claims 1 or 2, wherein the dry form is a lyophilisate.

4. The composition according to one of claims 1 - 3, wherein the vegetable extract is selected from extracts from fruit concentrate, herbal extracts and plant oils.

5. The composition according to one of claims 1 - 4, wherein the vegetable active substance or vegetable extract is selected from Aloe (Aloe vera), Rooibos (*Aspalathus linnearis*)*,* Guarana (*Paullinia cupana*)*,* white tea, green tea, black tea, hibiscus, nettle (*urticaceae*)*, Rorippa amphibia,* water cress, horse tail, field horsetail, chamomile, *Centella asiatica* (Gotu Kola), Gingko, Ginseng, cranberry (*Vaccinium macrocarpon*)*,* olive and pomegranate or mixtures thereof and/or extracts thereof.

6. A method for the production of a composition according to one of claims 1 - 5, **characterized in that** the components are dissolved or dispersed and homogenized in water, then preferably sterile filtered and subsequently freeze dried or vacuum-dried at - 30° C to +45° C, and the dried product is ground to a grain size with an orifice diameter of 0.5-1.5 mm.

7. The use of compositions according to one of claims 1 - 5 for the production of cosmetic, dietary or pharmaceutical formulations.

## Revendications

1. Composition à base de substance active végétale ou d'extrait végétal, de maltodextrine et phospholipide, **caractérisée en ce que** la composition se trouve sous forme sèche en tant que poudre ou produit granulé et **en ce que** sont contenus
a. 28 - 32 % de substance active végétale ou d'extrait végétal,
b. 60 - 70 % de maltodextrine et
c. 2 - 8 % de phospholipide constitué de fractions phospholipidiques ayant une teneur en phosphatidylcholines de 40-80 %.

2. Composition selon la revendication 1, **caractérisée en ce que** sont contenus
a. 30 % de substance active végétale ou d'extrait végétal,
b. 67 % de maltodextrine et
c. 3 % de phospholipide.

3. Composition selon la revendication 1 ou 2, **caractérisée en ce que** la forme sèche est un lyophilisat.

4. Composition selon l'une quelconque des revendications 1 à 3, **caractérisée en ce que** comme extrait végétal sont prévus un extrait de concentrés de fruits, des extraits d'herbes ou des huiles végétales.

5. Composition selon l'une quelconque des revendications 14, **caractérisée en ce que** comme substances actives végétales ou extraits végétaux sont prévus l'aloès (*Aloe vera*)*,* le rooibos (*Aspalathus linnearis*)*,* le guarana (*Paullinia cupana*)*,* le thé blanc, le thé vert, le thé noir, l'hibiscus, l'ortie (*Urticaceae*) le cresson d'eau, le cresson de fontaine, la prêle, la prêle des champs, la camomille, *Centela asiatica* (Gotu kola), le ginkgo, le ginseng, la canneberge (cranberry, airelle, *Vaccinium macrocarpon*)*,* l'olive, la grenade ou des mélanges et/ou extraits de ceux-ci.

6. Procédé pour la préparation de compositions selon l'une quelconque des revendications 1 à 5, **caractérisé en ce qu'**on dissout ou disperse et homogénéise les composants dans de l'eau, puis de préférence on les stérilise par filtration et ensuite on les sèche sous vide ou lyophilise à une température de -30 °C à +45 °C et on broie le produit séché jusqu'à une granulométrie à un diamètre de trous de 0,5 - 1,5 mm.

7. Utilisation des compositions selon l'une quelconque des revendications 1 à 5, pour la fabrication de préparations cosmétiques, diététiques ou pharmaceutiques.
